# EUROPEAN PATENT SPECIFICATION

(11) **EP 3 797 718 B1**
(45) Date of publication and mention of the grant of the patent: **08.05.2024**
(21) Application number: 20756717.3
(22) Date of filing: 04.02.2020
(51) Int. Cl.: A61B 17/3205, A61B 17/12, A61B 17/00, A61B 90/00, A61B 17/30

(54) **REUSABLE ANORECTAL LIGATION DEVICE**
WIEDERVERWENDBARE ANOREKTALE LIGATURVORRICHTUNG
DISPOSITIF DE LIGATURE ANORECTALE RÉUTILISABLE

(30) Priority: 14.02.2019 CN 201910114850; 14.02.2019 CN 201920197443 U
(43) Date of publication of application: 31.03.2021
(73) Proprietor: Henan Tuoren Medical Technology Co., Ltd., Nanpu Subdistrict Office Changyuan Henan 453400 (CN)
(72) Inventor: WANG, Qiang, Henan 453400 (CN); GUO, Guohong, Henan 453400 (CN); WANG, Ruixia, Henan 453400 (CN); HOU, Xiaofei, Henan 453400 (CN); WANG, Shugang, Henan 453400 (CN); XU, Zhenfeng, Henan 453400 (CN); YU, Bin, Henan 453400 (CN); LI, Zhengyang, Henan 453400 (CN); CHENG, Wensheng, Henan 453400 (CN)
(74) Representative: Nordmeyer, Philipp Werner
(86) International application number: PCT/CN2020/074257
(87) International publication number: WO 2020/164409

(56) References cited:
- EP-A1- 2 792 317
- CN-A- 105 030 294
- CN-A- 105 662 520
- CN-A- 105 662 520
- CN-A- 106 175 883
- CN-A- 109 620 338
- CN-A- 109 620 339
- CN-A- 109 620 341
- CN-A- 109 620 342
- CN-A- 109 646 070
- CN-B- 105 232 107
- CN-B- 106 175 883
- CN-U- 203 988 202
- CN-U- 204 744 295
- CN-U- 209 770 459
- CN-U- 209 789 927
- CN-U- 209 789 929
- CN-U- 209 789 931
- CN-U- 209 789 934
- US-A1- 2010 234 949
- US-A1- 2014 058 410
- US-A1- 2014 058 410
- US-A1- 2016 220 258

## Description

The present application claims priorities to the following two Chinese patent applications,
1) Chinese Patent Application No. 201910114850.7, titled " REUSABLE ANORECTAL LIGATION DEVICE" , filed with the China National Intellectual Property Administration on February 14, 2019, and
2) Chinese Patent Application No. 201920197443.2, titled "REUSABLE ANORECTAL LIGATION DEVICE", filed with the China National Intellectual Property Administration on February 14, 2019.

### FIELD

The present application relates to the technical field of medical instruments, and in particular to a reusable anorectal ligation device.

### BACKGROUND

A common method for treating hemorrhoids is ligation, the principle of which is to tie a special rubber band onto a root or base of hemorrhoids, and use the elastic retraction force of rubber band to block the blood supply of the hemorrhoids, causing the hemorrhoids to necrose, atrophy, and fall off so as to achieve healing purpose. At present, the instruments used for ligation operation generally adopt a ligation device, which is absorbed onto the hemorrhoids by vacuum, and then clamps the hemorrhoids by an elastic wire, so as to achieve the purpose of necrosis of hemorrhoids.

Most of the existing ligation devices in the market are in the form of a hand-held gun, which are of a disposable product. However, under the requirement of low cost and low consumption of medical appliances, the previous situation that one patient used a set of therapeutic appliances was not conducive to reducing medical costs. Meanwhile, most of the existing ligation devices form negative pressure through an external vacuum pump, thus, when the ligation device is held, it is necessary to operate the external vacuum pump and pay attention to the operation of the vacuum pump, which is not only troublesome, but also distracts doctors' attention, besides, the operation time is long, which is not conducive to the smooth progress of the operation.

The patent with the patent number 201610036268X, tiled "REUSABLE ANORECTAL LIGATION DEVICE" can disassemble and assemble the straw and push rod part. A patient corresponds to a set of detachable straws and push rods, so that the ligation device can be reused, which may be convenient for medical staff to use and reduce medical costs; meanwhile, in order to prevent human tissues from being sucked into the vacuum pump in the process of sucking negative pressure, a storage tank is set between the vacuum pump and the straw.

In the above technical solution:
1) There is no set of structure to prevent the same set of straw and push rod from being reused. If the medical staff are in a hurry and do not replace the straw and push rod, there may occur cross-infection of germs, which is not conducive to the treatment and rehabilitation of patients;
2) The storage tank needs to be cleaned. If it is not cleaned or fails to achieve the disinfection effect required by medical treatment, it is easy to breed bacteria, which is not conducive to repeated use.

CN105662520A discloses a loop ligature device capable of continuously launching negative pressure to suck a haemorrhoid. The loop ligature device comprises a loop ligature device shell, a fixing tube, a pushing tube, a launching trigger and a gas inlet valve trigger. A cylinder is arranged in a handle of the loop ligature device shell, and a cylinder body of the cylinder and the loop ligature device shell are fixed. The bottom of a piston is connected with the gas inlet valve trigger through a movable pulling rod. A gas inlet one-way valve and a gas outlet one-way valve are arranged at the gas outlet of the cylinder. The gas inlet one-way valve is communicated with the fixing tube through a pipeline. A deflation valve is arranged on the pipeline between the gas inlet one-way valve and the fixing tube. A fixing threaded rod is arranged on the head of the fixing tube, a pushing threaded rod is arranged on the head of the pushing tube, a plurality of sliding grooves are evenly formed in the fixing threaded rod, and the pushing threaded rod is embedded into the sliding grooves of the fixing threaded rod. US2016/220258A1 provides a method for setting elastic threads in a ligation device and an automatic elastic thread ligation device using the method. At least one elastic thread is arranged along the outer wall of a barrel of the ligation device, and an annular sleeve with an adjustable aperture and formed at the front end of the elastic thread is sleeved on the outer wall of the front end of the barrel; a traction thread on the ligation device is connected with the annular sleeve of the elastic thread, and when the traction thread is pulled, the traction thread drives the annular sleeve to move towards the orifice of the barrel, until the annular sleeve is disengaged from the orifice; a force bearing part of the annular sleeve at the front end of the elastic thread is propped forwards, meanwhile, the tail end of the elastic thread is pulled backwards, and the countertraction between the two acting forces leads to a gradual decrease of the aperture of the annular sleeve at the front end of the elastic thread.

### SUMMARY

In order to solve the defects in the prior art, the present application provides a reusable anorectal ligation device, which can prevent medical staff from reusing a ligation mechanism as a disposable consumable, avoid infection and is safe to operate.

The technical solution adopted by the present application to solve the above-mentioned problems is to provide a reusable anorectal ligation device according to the invention as defined in claim 1. Preferred embodiments are defined in the dependent claims.

The reusable anorectal ligation device has an overall shape of a gun, including a handle and a ligation mechanism, the ligation mechanism is detachably connected to the handle, and a firing mechanism for firing the ligation mechanism and a suction mechanism for providing negative pressure for the ligation mechanism are arranged in the handle;
the ligation mechanism includes an inner barrel, a pushing sleeve and a thread tightening component for ligating focus tissue, and the pushing sleeve is sleeved on the inner barrel and may be displaced relative to the inner barrel, and an anti-reset structure for preventing the pushing sleeve from resetting after displacement is arranged between the pushing sleeve and the inner barrel, at least one thread tightening component is arranged along the outer wall of the pushing sleeve, and the thread tightening component is mounted on the pushing sleeve;
the firing mechanism includes a firing switch, a guiding push block and a vent pipe top seat, the vent pipe top seat is fixedly connected in the handle, the guiding push block is slidably connected on the vent pipe top seat, and the firing switch is rotationally connected on the handle and in transmission connection with the guiding push block, and an extension spring is arranged between the guiding push block and the vent pipe top seat, and the inner barrel is detachably connected to the vent pipe top seat;
the suction mechanism includes a four-way pipe, a negative pressure pump and a pressure relief component for relieving the pressure of the ligation mechanism, and a first port, a second port, a third port and a fourth port are provided at the four-way pipe, and the first port is communicated with the vent pipe top seat, the second port is communicated with the suction port of the negative pressure pump through a negative pressure pump connecting conduit, the third port is provided with the pressure relief component, and the fourth port is connected with a sensor connecting conduit.

An electric control device for controlling the negative pressure pump is further arranged in the handle.

One end of the inner barrel is a suction nozzle, and the other end of the inner barrel is a socket. It is defined that the position of the suction nozzle is front and the position of the socket is rear. The socket is fixedly connected to a rear side of the suction nozzle. A clamping part is arranged between the suction nozzle and the socket, the clamping part is close to one side of the socket, an even number of guide grooves are symmetrically provided on the clamping part, which run through from front to back. Each guide groove is slidably connected with a pushing sleeve limit block, a third clamping groove is provided at the bottom of the guide groove, and an even number of first buckles are symmetrically provided on the clamping part.

The anti-reset structure is configured such that: an even number of second buckles are symmetrically provided on a tail end of the pushing sleeve , and a second clamping groove is provided at one side of the pushing sleeve limit block close to the axis of the inner barrel. The second buckle is clamped in the second clamping groove, and a third buckle is arranged on one side, far away from the axis of the inner barrel, of the pushing sleeve limit block. Before the pushing sleeve is displaced relative to the inner barrel, the third buckle is located in the second clamping groove; after the pushing sleeve is displaced relative to the inner barrel, the third buckle is clamped in the third clamping groove.

Preferably, the pressure relief component includes a pressure relief key, a pressure relief cover and a compression spring. The pressure relief key extends out of one end of the third port and is slidably connected in the third port. The pressure relief cover is threadedly

Preferably, the exhaust port of the negative pressure pump is connected with an exhaust conduit, an exhaust joint is mounted on the exhaust conduit, and a check valve is mounted in the exhaust joint; the exhaust joint is clamped at the bottom of the handle, and an exhaust joint sealing ring is mounted between the exhaust joint and the handle; a damping sleeve covers an exterior of the negative pressure pump.

Preferably, a battery and the electric control device are mounted inside the handle. A battery compartment is arranged in the handle, and the battery is detachably mounted in the battery compartment. The battery is electrically connected with the electric control device and the negative pressure pump respectively, and the negative pressure pump is arranged below the battery. The electric control device is electrically connected with a pressure sensor, and the probe of the pressure sensor is arranged in the sensor connecting conduit. One end of the sensor connecting conduit is closed, and the other end of the sensor connecting conduit is communicated with a four-way pipe. The negative pressure pump is connected with a power key through wires, and the power key is mounted on the handle; the electric control device is further connected with a display screen, and the display screen is mounted on the handle.

Preferably, the vent pipe top seat includes a barrel connecting part and a pipeline connecting part. The barrel connecting part and the pipeline connecting part are mutually communicated by respective negative pressure channels, the barrel connecting part and the inner barrel are coaxially arranged, a tail end of the inner barrel is inserted into the barrel connecting part, and the pipeline connecting part is communicated with the suction mechanism.
connected with the other end of the third port, and the compression spring is arranged between the pressure relief key and the pressure relief cover. A pressure relief key sealing ring is mounted between the pressure relief key and the third port, and a pressure relief cover sealing ring is mounted between the pressure relief cover and the third port.
in the present application is arranged below the battery, so that the handle structure is more compact and the handle volume is reduced. Meanwhile, the negative pressure pump and battery are integrated inside the handle, and there is no need to pay attention to the operation of external vacuum pump, which avoids distracting medical staff, and is convenient to operate, which is conducive to the smooth progress of the operation.

The dustproof protective cover of the present application is in interference fit with the charging connector, and each sealing ring is in interference fit with the corresponding holes on the left handle shell and the right handle shell. They also form a closed space with the L-shaped retaining wall inside the handle, which effectively protects the electric control device, battery and negative pressure pump from external bacteria and sterilization. After a case is completed, press the dust-proof cover in place to perform disinfection and sterilization, and prepare to reuse the handle next time.

The present application can prevent medical staff from reusing the ligation mechanism as a consumable, can avoid infection, and has safe operation.

### BRIEF DESCRIPTION OF THE DRAWINGS

Figure 1 is a first schematic diagram of a three-dimensional structure inside a handle of the present application;
Figure 2 is a second schematic diagram of the three-dimensional structure inside the handle of the present application;
Figure 3 is a schematic diagram of an external structure of the present application;
Figure 4 is an exploded schematic diagram of parts of some structures in the present application;
Figure 5 is a partially enlarged schematic diagram at A in Figure 2;
Figure 6 is a partially enlarged schematic diagram at B in Figure 2;
Figure 7 is a schematic diagram of a three-dimensional structure of a ligation mechanism of the present application;
Figure 8 is an exploded schematic diagram of the parts of Figure 7;
Figure 9 is a schematic plan view of the ligation mechanism and a firing mechanism of the present application;
Figure 10 is a schematic cross-sectional view of the structure of Figure 9 in a top view;
Figure 11 is a partial sectional view of the structure of a pushing sleeve before moving in the present application;
Figure 12 is a partial sectional view of the structure of the pushing sleeve after moving in the present application;
Figure 13 is a partially enlarged schematic diagram at C in Figure 12;

### Reference numerals are as follows:

| | | | |
|---|---|---|---|
| 11 | left handle shell | 12 | right handle shell |
| 13 | left release key | 14 | right release key |
| 15 | L-shaped retaining wall | 16 | battery compartment |
| 17 | dustproof protective cover | 18 | light transmission piece |
| 21 | inner barrel | 22 | pushing sleeve |
| 23 | thread tightening component | 211 | suction nozzle |
| 212 | socket | 213 | clamping part |
| 214 | guide groove | 215 | pushing sleeve limit block |
| 216 | third clamping groove | 217 | first buckle |
| 218 | inner barrel sealing ring | 221 | second buckle |
| 222 | second clamping groove | 223 | third buckle |
| 224 | limit ring | 232 | tightening pipe |
| 233 | tightening cap | 234 | tightening clamping joint |
| 235 | ring sleeve | 236 | wire knot |
| 237 | fixing ring | 238 | clamping position |
| 239 | first non-slip edge | 31 | firing switch |
| 32 | guiding push block | 33 | vent pipe top seat |
| 34 | extension spring | 321 | guiding cavity |
| 322 | push rod | 323 | transmission block |
| 324 | limit step | 331 | barrel connecting part |
| 332 | pipeline connecting part | 334 | guide rail |
| 335 | first clamping groove | 336 | vent seat gasket |
| 337 | vent seat sealing ring | 41 | four-way pipe |
| 42 | negative pressure pump | 43 | pressure relief component |
| 44 | negative pressure pump connecting conduit | | |
| 45 | sensor connecting conduit | | |
| 421 | exhaust conduit | 422 | exhaust joint |
| 423 | check valve | 424 | exhaust joint sealing ring |
| 425 | damping sleeve | 431 | pressure relief key |
| 432 | pressure relief cover | 433 | compression spring |
| 434 | pressure relief key sealing ring | 435 | pressure relief cover sealing ring |
| 51 | battery | 52 | power key |
| 54 | display screen | | |

### DETAILED DESCRIPTION

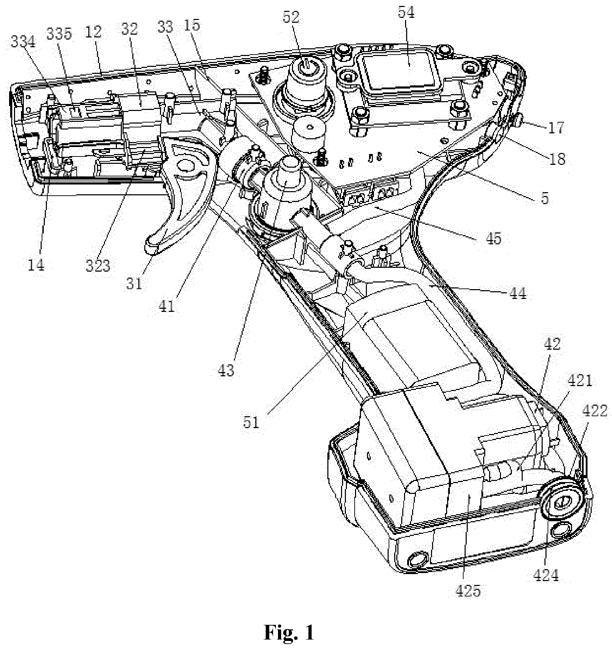

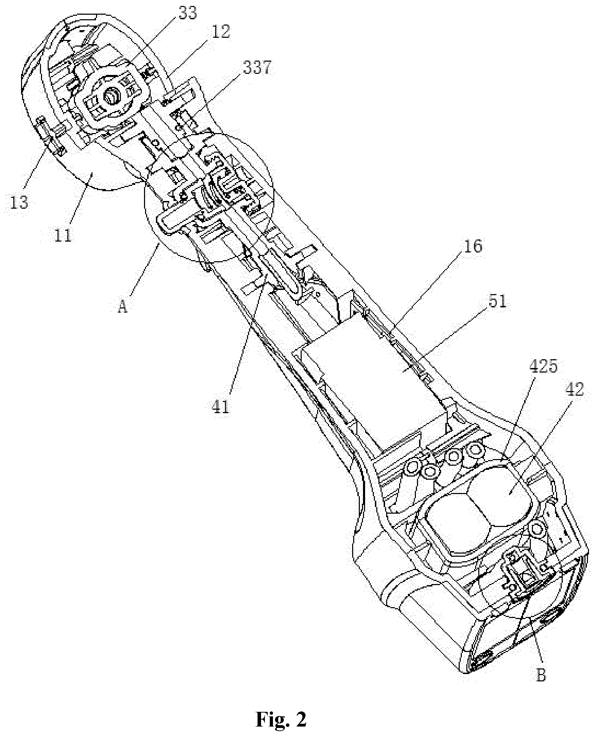

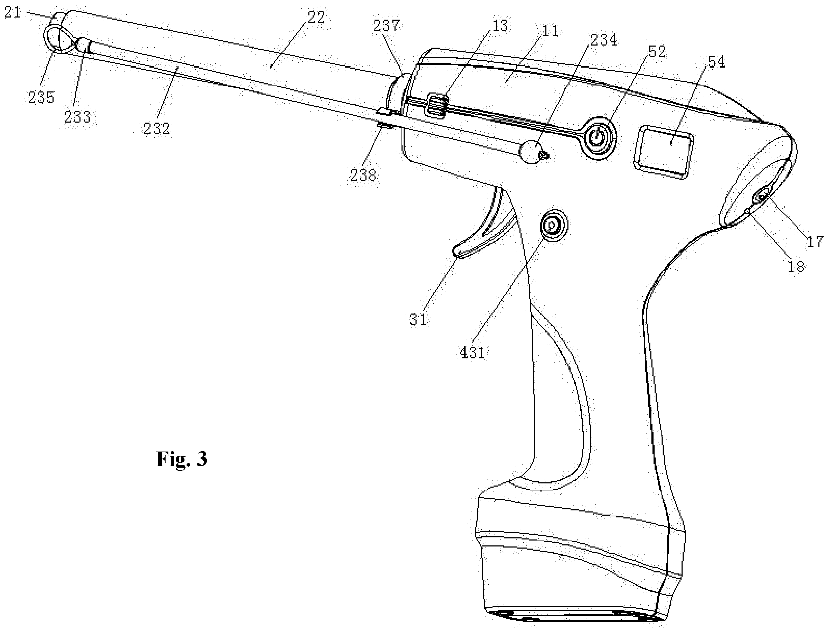

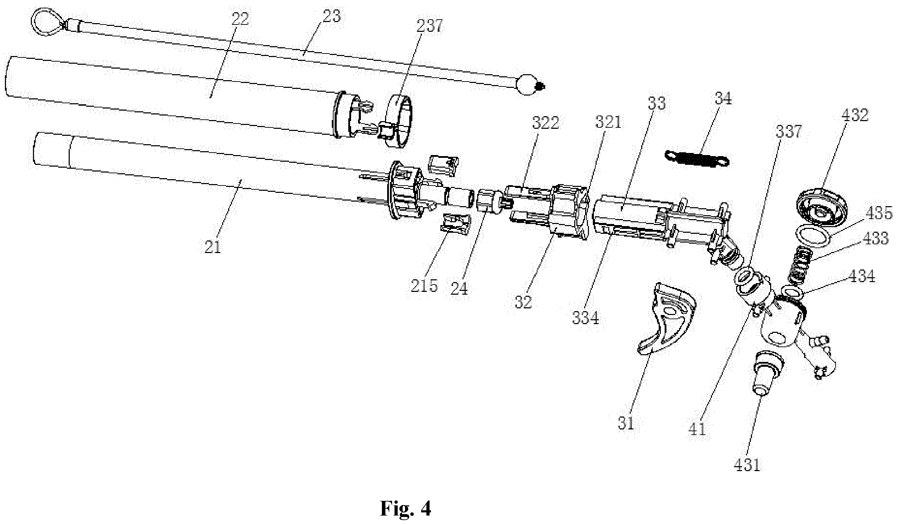

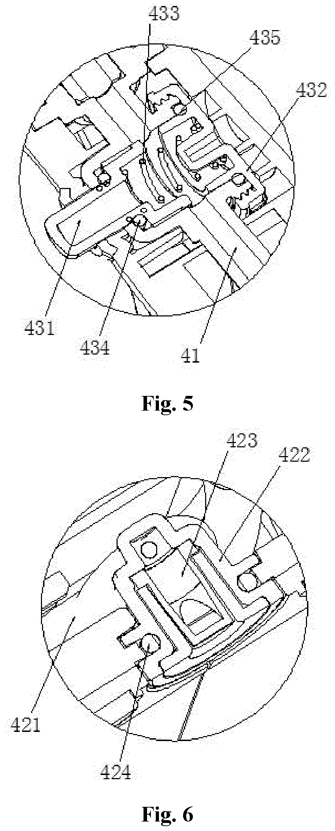

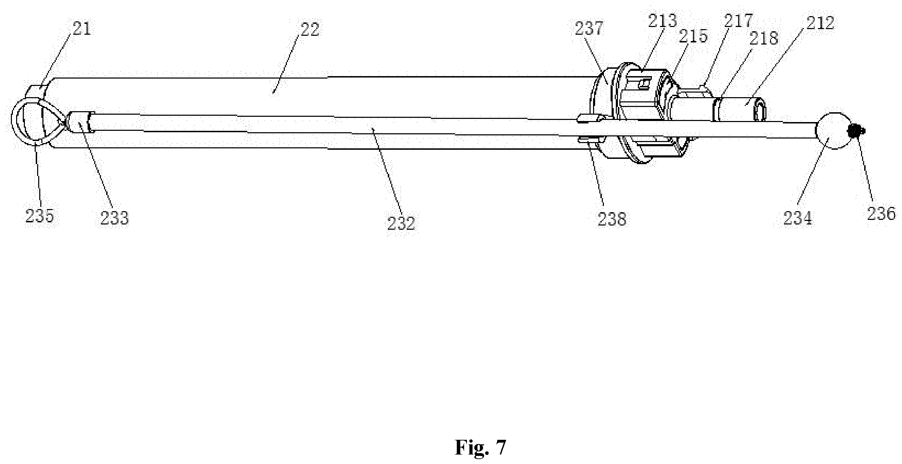

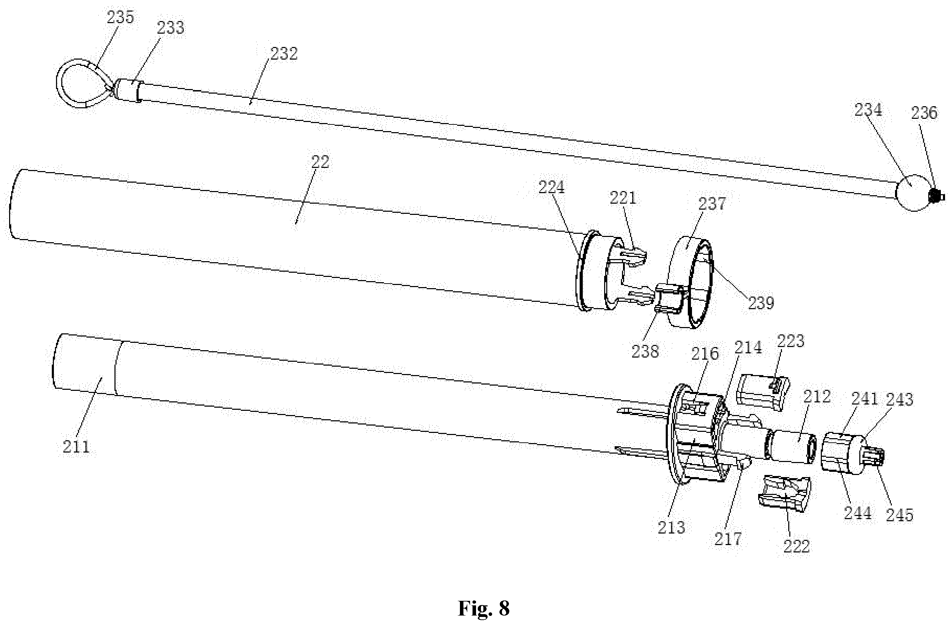

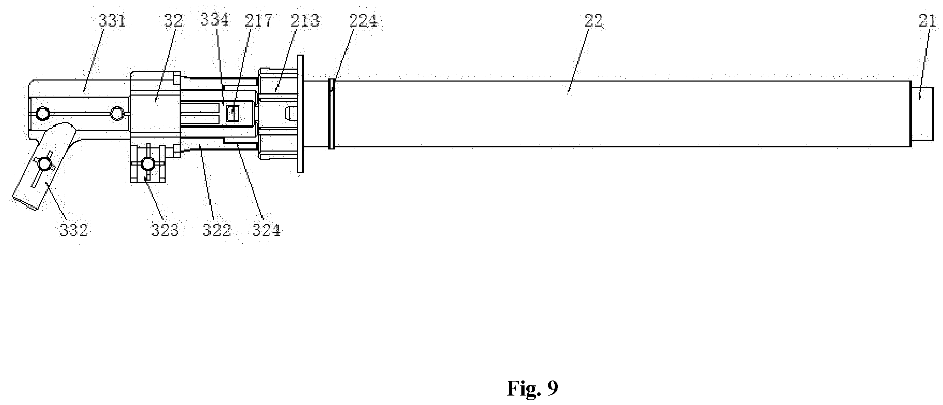

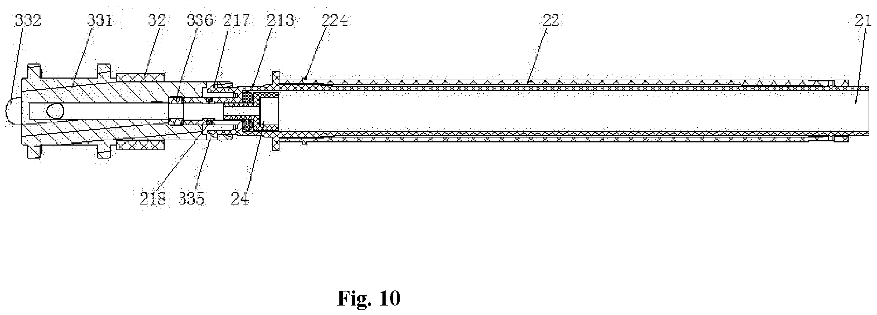

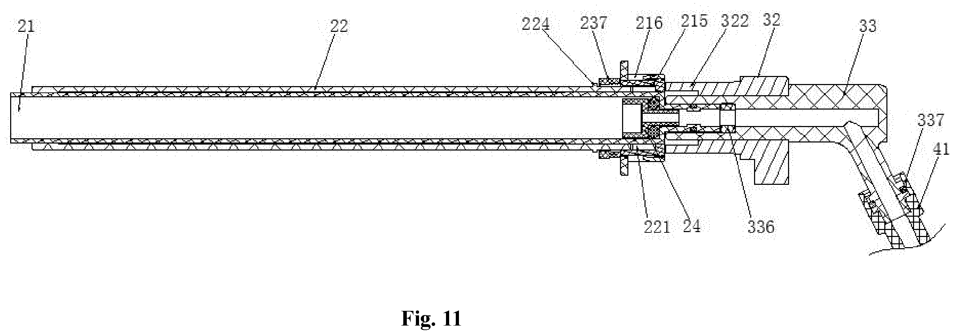

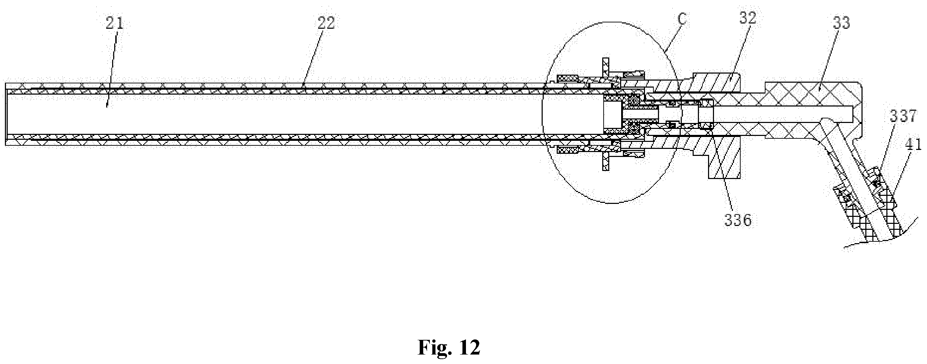

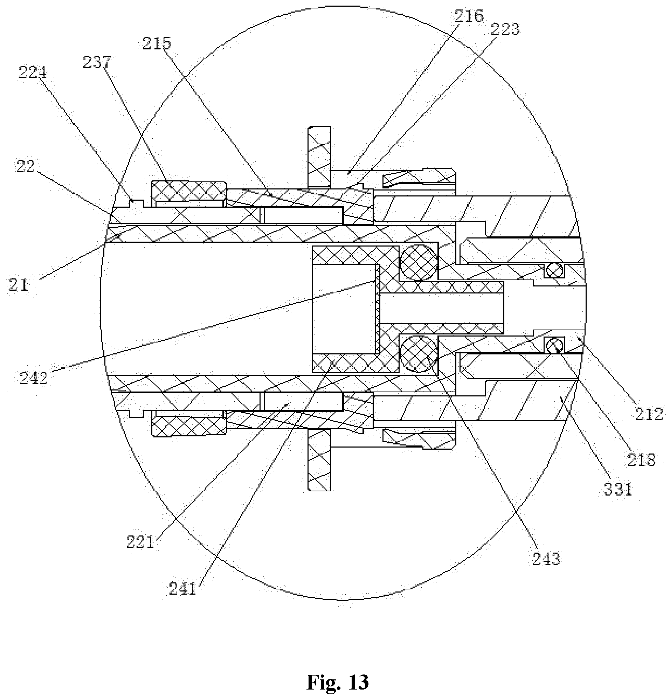

As shown in Figure 1 to Figure 13, a reusable anorectal ligation device according to the present application is gun-shaped, including a handle and a ligation mechanism. The ligation mechanism is detachably connected to the handle. The handle includes a left handle shell 11 and a right handle shell 12, and the left handle shell 11 and the right handle shell 12 are buckled and assembled with each other to form a gun shell, and a firing mechanism for firing the ligation mechanism and a suction mechanism for providing negative pressure for the ligation mechanism are arranged in the handle.

The ligation mechanism includes an inner barrel 21, a pushing sleeve 22 and a thread tightening component 23 for ligating focus tissue. The pushing sleeve 22 is sleeved on the inner barrel 21 and can be displaced relative to the inner barrel 21. An anti-reset structure for preventing the pushing sleeve 22 from resetting after displacement is arranged between the pushing sleeve 22 and the inner barrel 21, at least one thread tightening component 23 is arranged along the outer wall of the pushing sleeve 22, and the thread tightening component 23 is mounted on the pushing sleeve 22.

A suction nozzle 211 is provided at one end of the inner barrel 21, and a socket 212 is provided at the other end of the inner barrel 21. The position of the suction nozzle 211 is considered to be front and the position of the socket 212 is considered to be rear. The socket 212 is fixedly connected to a rear side of the suction nozzle 211. A clamping part 213 is arranged between the suction nozzle 211 and the socket 212, the clamping part 213 is close to one side of the socket 212, an even number of guide grooves 214, which run through from front to back, are symmetrically provided on the clamping part 213. Each guide groove 214 is slidably connected with a pushing sleeve limit block 215, a third clamping groove 216 is provided at the bottom of the guide groove 214, and an even number of first buckles 217 are symmetrically provided on the clamping part 213. The clamping part 213 is detachably connected with the firing mechanism through the two first buckles 217.

The specific structure of the anti-reset structure is that an even number of second buckles 221 are symmetrically provided at the tail end of the pushing sleeve 22, and a second clamping groove 222 is provided at one side, close to the axis of the inner barrel 21, of the pushing sleeve limit block 215. The second buckles 221 are respectively clamped in the second clamping grooves 222, and a third buckle 223 is arranged on one side, far away from the axis of the inner barrel 21, of the pushing sleeve limit block 215. Before the pushing sleeve 22 is displaced relative to the inner barrel 21, the third buckle 223 is located in the second clamping groove 222; after the pushing sleeve 22 is displaced relative to the inner barrel 21, the third buckle 223 is clamped in the third clamping groove 216. When one push action is completed, the pushing sleeve limit block 215 is limited by the third clamping groove 216 on the clamping part 213 of the inner barrel 21 and cannot return. The ligation mechanism of the present application can be prevented from being repeatedly used, and the requirements for disposable use of consumables can be met.

An inner barrel sealing ring 218 is provided on the socket 212 of the inner barrel 21, which plays a sealing role when the socket 212 is detachably connected to the matching handle, and keeps the tightness in working condition.

The thread tightening component 23 includes an elastic wire, a tightening pipe 232, a tightening cap 233 and a tightening clamping joint 234. A ring sleeve 235 with adjustable aperture is provided at the front end of the elastic wire (the front end of the elastic wire wraps around a ferrule and then forms a slipknot with the elastic wire itself), a wire knot 236 is provided at the rear end of the elastic wire, and the middle part of the elastic wire passes through the tightening pipe 232. A tightening cap 233 is provided at the front end of the tightening pipe 232, a tightening clamping joint 234 is provided at the rear end of the tightening pipe 232, the ring sleeve 235 is limited by the tightening cap 233, and the wire knot 236 is limited by the tightening clamping joint; the ring sleeve 235 is sleeved on the inner barrel 21 and is limited at the front end face of the pushing sleeve 22. When the rear end of the elastic wire is pulled backward, the aperture of the ring sleeve 235 at the front end of the elastic wire is gradually reduced.

The inner diameter of the pushing sleeve 22 is matched with the outer diameter of the inner barrel 21. When the pushing sleeve 22 is displaced relative to the inner barrel 21, the front end face of the pushing sleeve 22 pushes out the ring sleeve 235 at the front end of the elastic wire, so that the ring sleeve 235 is tied at the root of the hemorrhoid.

The thread tightening component 23 further includes a fixing ring 237 mounted on the pushing sleeve 22, and the limit ring 224 is provided at the rear part of the pushing sleeve 22, and the fixing ring 237 is mounted between the limit ring 224 and the clamping part 213 of the inner barrel 21. A clamping groove 238 is provided on the fixing ring 237, and the tail of the tightening pipe 232 is mounted in the clamping groove 238.

Multiple first non-slip edges 239 are provided on the inner wall of the fixing ring 237, which are evenly spaced along the circumferential direction, so as to prevent the fixing ring 237 from rotating on the pushing sleeve 22.

The firing mechanism includes a firing switch 31, a guiding push block 32 and a vent pipe top seat 33. The vent pipe top seat 33 is fixedly connected in the handle, the guiding push block 32 is slidably connected on the vent pipe top seat 33, and the firing switch 31 (trigger) is rotationally connected on the handle and in transmission connection with the lower end of the guiding push block 32. An extension spring 34 is arranged between the guiding push block 32 and the vent pipe top seat 33, which is used for reseting the firing switch 31 after the pushing sleeve 22 is displaced, and the inner barrel 21 is detachably connected to the vent pipe top seat 33.

The vent pipe top seat 33 includes a barrel connecting part 331 and a pipeline connecting part 332. The barrel connecting part 331 and the pipeline connecting part 332 are mutually communicated by their respective negative pressure channels, the barrel connecting part 331 and the inner barrel 21 are coaxially arranged, a tail end of the inner barrel 21 is inserted into the barrel connecting part 331, and the pipeline connecting part 332 is communicated with the suction mechanism.

An even number of guide rails 334 are symmetrically provided at two sides of the barrel connecting part 331, a first clamping groove 335 are provided on each guide rail 334, and the first buckles 217 on the clamping part 213 of the inner barrel 21 are clamped in the first clamping groove 335. The guide rails 334 are provided to limit the guiding push block 32 to prevent its misalignment from being unable to push the pushing sleeve 22.

The handle is equipped with a left release key 13 and a right release key 14. The left release key 13 and the right release key 14 are respectively matched with the first buckle 217 on the clamping port 213 of the inner barrel 21; the left release key 13 and the right release key 14 have the same structure. The left release key 13 includes a pressing part which projects from the handle and is suspended, one end of the pressing part is connected with a rebound part. The rebound part is fixed on the inner wall of the handle. An L-shaped retaining wall 15 is arranged in the handle, which effectively isolates the negative pressure pump 42 from the electric control device and avoids the influence of external bacteria and sterilization.

A vent seat gasket 336 is mounted between the tail end of the inner barrel 21 and the barrel connecting part 331, and a vent seat sealing ring 337 is mounted on the pipeline connecting part 332.

A guiding cavity 321 penetrating back and forth is provided at the guiding push block 32, and the guiding push block 32 is slidably connected to the vent pipe top seat 33 through the guiding cavity 321. An even number of push rods 322, matching with the pushing sleeve limit blocks 215, are symmetrically provided at the front end of the guiding push block 32. A transmission block 323 contacting with the firing switch 31 is provided at the lower part of the guiding push block 32.

A limit step 324 is provided on the push rod 322, the length of the limit step 324 is smaller than the length of the pushing sleeve limit block 215, and the length of the pushing sleeve limit block 215 is larger than the displacement stroke of the pushing sleeve 22 to prevent the push action from failing.

The suction mechanism includes a four-way pipe 41, a negative pressure pump 42 and a pressure relief component 43 for relieving the pressure of the ligation mechanism. A first port, a second port, a third port, and a fourth port are provided on the four-way pipe 41. The first port is communicated with the vent pipe top seat 33, the second port is communicated with the suction port of the negative pressure pump 42 through a negative pressure pump connecting conduit 44, the third port is provided with the pressure relief component 43, and the fourth port is connected with a sensor connecting conduit 45; the inner barrel 21, the vent pipe top seat 33, the four-way pipe 41, the negative pressure pump connecting conduit 44 and the negative pump 42 are sequentially communicated to form a negative pressure pumping channel.

The third port and a main pipe of the four-way pipe 41 are arranged in a cross shape. The pressure relief component 43 includes a pressure relief key 431, a pressure relief cover 432 and a compression spring 433. The pressure relief key 431 extends out of one end of the third port and is slidably connected in the third port. The pressure relief cover 432 is threadedly connected with the other end of the third port, and the compression spring 433 is arranged between the pressure relief key 431 and the pressure relief cover 432. By pressing the compression spring 433 with the pressure relief key 431, one end of the third port may be opened for pressure relief. A pressure relief key sealing ring 434 is mounted between the pressure relief key 431 and the third port, and a pressure relief cover sealing ring 435 is mounted between the pressure relief cover 432 and the third port.

An exhaust port of the negative pressure pump 42 is connected with an exhaust conduit 421, an exhaust joint 422 is mounted on the exhaust conduit 421, and a check valve 423 is mounted in the exhaust joint 422, the check valve 423 may be a duckbill valve; the exhaust joint 422 is clamped at the bottom of the handle, and an exhaust joint sealing ring 424 is mounted between the exhaust joint 422 and the handle; the outer circumference of the negative pressure pump 42 is covered with a damping sleeve 425. The negative pressure pump 42 can be coated with silicone sleeve, which can reduce vibration and noise.

An electric control device 5 for controlling the negative pressure pump 42 is further provided in the handle. The electric control device may be a PLC, an integrated circuit or a single-chip microcomputer. The model of MCU may be MSP430G2332.

A battery 51 and the electric control device are mounted inside the handle. A battery compartment 16 is arranged in the handle, and the battery 51 is detachably mounted in the battery compartment 16. The battery 51 is electrically connected with the electric control device and the negative pressure pump 42 respectively, and the negative pressure pump 42 is arranged below the battery 51. The electric control device is electrically connected with a pressure sensor, and a probe of the pressure sensor is arranged in the sensor connecting conduit 45. One end of the sensor connecting conduit 45 is closed, and the other end of the sensor connecting conduit 45 is communicated with a four-way pipe. The negative pressure pump 42 is connected with a power key 52 through wires, and the power key 52 is mounted on the handle; the electric control device is further connected with a display screen 54, where the display screen 54 is mounted on the handle.

In specific application, the electric control device 5 may be a PCB board, and the PCB board is fixed inside the handle. A pressure sensor, a display screen, a self-locking switch, a patch LED, a buzzer and a charging connector are mounted on the PCB board; a charging port and a light transmission port are provided on the handle, a dustproof protective cover 17 is provided on the charging port, and the dustproof protective cover is a flip cover. The charging connector may be exposed if the dustproof protective cover is opened, and after the dustproof protective cover is fastened on the handle, it may cover up the charging connector; and a light transmission piece 18 is provided at the light transmission port, which is convenient for the patch LED to transmit light.

The dustproof protective cover 17 is in interference fit with the charging connector, and each sealing ring is in interference fit with the corresponding holes on the left handle shell 11 and the right handle shell 12. They also form a closed space with the L-shaped retaining wall 15 inside the handle, which effectively protects the electric control device 5, battery 15 and negative pressure pump 42 from external bacteria and sterilization. After a case is completed, press the dust-proof cover in place to perform disinfection and sterilization, and prepare to reuse the handle next time.

During the operation, the ligation mechanism is mounted into the first clamping groove 335 of the vent pipe top seat 33 inside the handle through the front end of the handle, and if the power key 52 is pressed once, the negative pressure pump 42 starts to work. Then, the user finds the part that needs to be tied, and when the negative pressure value on the display screen 54 is between -0.08MPa ~ -0.1 OMPa, the user pulls the firing switch 31. The guiding push block 32 pushes the pushing sleeve limit block 215 and the pushing sleeve 22 under the action of the firing switch 31, and pushes the ring sleeve 235 of the elastic wire to the position where ligation is required. The elastic wire is tightened by the tightening clamping joint 234 and the wire knot 236 at one end of the tightening pipe, then the elastic wire is cut off, and the tightening device is taken out. Next, the pressure relief key is pressed to relieve the pressure, the handle is exited, the power key 52 is pressed once to turn off the power, and the ligation of the part is completed.

When one push action is completed, the position of the pushing sleeve limit block 215 is limited by the two third buckles 2233 at one end of the inner barrel 21, and cannot return, and the ligation mechanism cannot be reused, which meets the requirement of disposable use of consumables. The left release key 13 and right release key 14 are pressed on the left and right handle shells, so as to make the ligation mechanism take out for preparing the next ligation action.

The present application can not only prevent medical staff from reusing the ligation mechanism as a disposable consumable, but also prevent foreign matter from entering the inside of the matched handle, so that infection can be avoided, and the operation is safe.

The negative pressure pump 42, elastic wire, motor, speed reducer, filter membrane 242, filter sealing ring 243, pressure sensor, display screen 54, self-locking switch, patch LED, buzzer and charging connector are conventional devices, and the specific structure will not be described in detail.

The above embodiments do not limit the shape, material and structure of the present patent in any form. The scope of the invention is supported by these embodiments but defined by the appended claims.

## Claims

1. A reusable anorectal ligation device having an overall shape of a gun, comprising a handle and a ligation mechanism, wherein, the ligation mechanism is detachably connected to the handle, and a firing mechanism for firing the ligation mechanism and a suction mechanism for providing negative pressure for the ligation mechanism are arranged in the handle; and
the ligation mechanism comprises an inner barrel (21), a pushing sleeve (22) and a thread tightening component (23) for ligating focus tissue, wherein the pushing sleeve (22) is sleeved on the inner barrel (21) and is configured to be displaced relative to the inner barrel (21), and an anti-reset structure for preventing the pushing sleeve (22) from resetting after displacement is arranged between the pushing sleeve (22) and the inner barrel (21), the at least one thread tightening component (23) is arranged along an outer wall of the pushing sleeve (22), and the thread tightening component (23) is mounted on the pushing sleeve (22); and
the firing mechanism comprises a firing switch (31), a guiding push block (32) and a vent pipe top seat (33), wherein the vent pipe top seat (33) is fixedly connected in the handle, the guiding push block (32) is slidably connected to the vent pipe top seat (33), and the firing switch (31) is rotationally connected on the handle and in transmission connection with the guiding push block (32); an extension spring (34) is arranged between the guiding push block (32) and the vent pipe top seat (33), and the inner barrel (21) is detachably connected to the vent pipe top seat (33);
the suction mechanism comprises a four-way pipe (41), a negative pressure pump (42) and a pressure relief component (43) for relieving the pressure of the ligation mechanism, wherein a first port, a second port, a third port and a fourth port are provided at the four-way pipe (41); and the first port is communicated with the vent pipe top seat (33), the second port is communicated with a suction port of the negative pressure pump (42) through a negative pressure pump connecting conduit (44), the third port is mounted with the pressure relief component (43), and the fourth port is connected with a sensor connecting conduit (45); and
an electric control device (5) for controlling the negative pressure pump (42) is further arranged in the handle,
wherein, one end of the inner barrel (21) is a suction nozzle (211), and the other end of the inner barrel (21) is a socket (212); and it is defined that a position of the suction nozzle (211) is front and a position of the socket (212) is rear; and
the socket (212) is fixedly connected to a rear side of the suction nozzle (211), a clamping part (213) is arranged between the suction nozzle (211) and the socket (212), the clamping part (213) is closer to the socket (212), an even number of guide grooves (214), which run through from front to back, is symmetrically provided on the clamping part (213); and
each guide groove (214) is slidably connected with a pushing sleeve limit block (215), a third clamping groove (216) is provided at the bottom of the guide groove (214), and an even number of first buckles (217) is symmetrically provided on the clamping part (213),
**characterized in that**, the anti-reset structure consist of :
an even number of second buckles (221) symmetrically provided on a tail end of the pushing sleeve (22), and a second clamping groove (222) provided at one side of the pushing sleeve limit block (215) close to an axis of the inner barrel (21); wherein
each second buckle (221) is clamped in the second clamping groove (222), and a third buckle (223) is arranged on the other side of the pushing sleeve limit block (215), the other side being far away from the axis of the inner barrel (21); and wherein
before the pushing sleeve (22) displaces relative to the inner barrel (21), the third buckle (223) is located in the second clamping groove (222), and after the pushing sleeve (22) has been displaced relative to the inner barrel (21), the third buckle (223) is clamped in the third clamping groove (216) such that the pushing sleeve (22) cannot return to the previous position.

2. The reusable anorectal ligation device according to claim 1, wherein, the pressure relief component comprises a pressure relief key (431), a pressure relief cover (432) and a compression spring (433); and
the pressure relief key (431) extends out of one end of the third port and is slidably connected in the third port; and
the pressure relief cover (432) is threadedly connected with the other end of the third port, and the compression spring (433) is arranged between the pressure relief key (431) and the pressure relief cover (432); and
a pressure relief key sealing ring (434) is mounted between the pressure relief key (431) and the third port, and a pressure relief cover sealing ring (435) is mounted between the pressure relief cover (432) and the third port.

3. The reusable anorectal ligation device according to claim 1, wherein, an exhaust port of the negative pressure pump (42) is connected with an exhaust conduit (421), an exhaust joint (422) is mounted on the exhaust conduit (421), and a check valve (423) is mounted in the exhaust joint (422); the exhaust joint (422) is clamped at the bottom of the handle, and an exhaust joint sealing ring (424) is mounted between the exhaust joint (422) and the handle; and a damping sleeve (425) covers an exterior of the negative pressure pump (42).

4. The reusable anorectal ligation device according to claim 1, wherein, a battery (51) and the electric control device (5) are mounted inside the handle, a battery compartment (16) is arranged in the handle, and the battery (51) is detachably mounted in the battery compartment (16); and
the battery (51) is electrically connected with the electric control device (5) and the negative pressure pump (42) respectively, and the electric control device (5) is electrically connected with a pressure sensor, and a probe of the pressure sensor is arranged in a sensor connecting conduit (45); and
one end of the sensor connecting conduit (45) is closed, and the other end of the sensor connecting conduit (45) is communicated with a four-way pipe; the negative pressure pump (42) is connected with a power key (52) through wires, and the power key (52) is mounted on the handle; the electric control device (5) is further connected with a display screen (54), and the display screen (54) is mounted on the handle.

5. The reusable anorectal ligation device according to claim 1, wherein, the vent pipe top seat (33) comprises a barrel connecting part (331) and a pipeline connecting part (332), and the barrel connecting part (331) and the pipeline connecting part (332) are mutually communicated by respective internal negative pressure channels, the barrel connecting part (331) and the inner barrel (21) are coaxially arranged, a tail end of the inner barrel (21) is inserted into the barrel connecting part (331), and the pipeline connecting part (332) is communicated with the suction mechanism; and
an even number of guide rails are symmetrically provided at two sides of the barrel connecting part (331), a first clamping groove (335) is provided on the each guide rail, and each first buckle (217) on the inner barrel clamping part (213) is clamped in a corresponding first clamping groove (335).

6. The reusable anorectal ligation device according to claim 5, wherein, a guiding cavity, penetrating back and forth, is provided at the guiding push block (32), and the guiding push block (32) is slidably connected to the vent pipe top seat (33) through the guiding cavity, an even number of push rods (322), matching with the pushing sleeve limit block (215), are symmetrically provided at a front end of the guiding push block (32); and
a transmission block, contacting with the firing switch (31), is provided at the lower part of the guiding push block (32), and a limit step is provided on each push rod (322).

7. The reusable anorectal ligation device according to claim 6, wherein, the handle is equipped with a left release key (13) and a right release key (14), the left release key (13) and the right release key (14) are respectively matched with the first buckle (217) on the inner barrel clamping part (213), and an L-shaped retaining wall (15) is provided in the handle.

8. The reusable anorectal ligation device according to claim 1, wherein, a charging port and a light transmission port are provided on the handle, a dustproof protective cover (17) is provided on the charging port, and a light transmission piece (18) which is convenient for the patch LED to transmit light is provided at the light transmission port.

## Patentansprüche

1. Wiederverwendbare anorektale Ligaturvorrichtung mit einer Gesamtform einer Pistole, umfassend einen Griff und einen Ligaturmechanismus, wobei der Ligaturmechanismus lösbar mit dem Griff verbunden ist und ein Auslösemechanismus zum Auslösen des Ligaturmechanismus und ein Saugmechanismus zum Bereitstellen von Unterdruck für den Ligaturmechanismus im Griff angeordnet sind; und
der Ligaturmechanismus einen Innenzylinder (21), eine Schiebehülse (22) und eine Fadenspannkomponente (23) zum Abbinden von Fokusgewebe umfasst, wobei die Schiebehülse (22) auf dem Innenzylinder (21) aufgesteckt und ausgestaltet ist, relativ zu dem Innenzylinder (21) verschoben zu werden, und eine Rückstellsperre zwischen der Schiebehülse (22) und dem Innenzylinder (21) angeordnet ist, um zu verhindern, dass sich die Schiebehülse (22) nach der Verschiebung zurückstellt, die mindestens eine Fadenspannkomponente (23) entlang einer Außenwand der Schiebehülse (22) angeordnet ist und die Fadenspannkomponente (23) an der Schiebehülse (22) montiert ist; und
der Auslösemechanismus einen Auslöseschalter (31), einen Führungsschubblock (32) und einen oberen Sitz (33) eines Entlüftungsrohrs umfasst, wobei der obere Sitz (33) des Entlüftungsrohrs fest in dem Griff verbunden ist, der Führungsschubblock (32) gleitfähig mit dem oberen Sitz (33) des Entlüftungsrohrs verbunden ist und der Auslöseschalter (31) drehbar auf dem Griff verbunden ist und in Übertragungsverbindung mit dem Führungsschubblock (32) steht; eine Zugfeder (34) zwischen dem Führungsschubblock (32) und dem oberen Sitz (33) des Entlüftungsrohrs angeordnet ist und der Innenzylinder (21) lösbar mit dem oberen Sitz (33) des Entlüftungsrohrs verbunden ist;
der Saugmechanismus ein Vierwegerohr (41), eine Unterdruckpumpe (42) und eine Druckentlastungskomponente (43) zur Druckentlastung des Ligaturmechanismus umfasst, wobei ein erster Anschluss, ein zweiter Anschluss, ein dritter Anschluss und ein vierter Anschluss an dem Vierwegerohr (41) vorgesehen sind; und
der erste Anschluss mit dem oberen Sitz (33) des Entlüftungsrohrs kommuniziert, der zweite Anschluss über eine Unterdruckpumpen-Verbindungsleitung (44) mit einem Ansauganschluss der Unterdruckpumpe (42) kommuniziert, der dritte Anschluss mit der Druckentlastungskomponente (43) montiert ist und der vierte Anschluss mit einer Sensorverbindungsleitung (45) verbunden ist; und
im Griff weiter eine elektrische Steuervorrichtung (5) zum Steuern der Unterdruckpumpe (42) angeordnet ist,
wobei ein Ende des Innenzylinders (21) eine Saugdüse (211) und das andere Ende des Innenzylinders (21) ein Stutzen (212) ist; und definiert ist, dass eine Position der Saugdüse (211) vorne und eine Position des Stutzens (212) hinten ist; und
der Stutzen (212) fest mit einer Rückseite der Saugdüse (211) verbunden ist, ein Klemmteil (213) zwischen der Saugdüse (211) und dem Stutzen (212) angeordnet ist, das Klemmteil (213) nahe an einer Seite des Stutzens (212) ist, am Klemmteil (213) symmetrisch eine gerade Anzahl von Führungsnuten (214), die von vorne nach hinten verlaufen, vorgesehen ist; und
jede Führungsnut (214) gleitfähig mit einem Schiebehülsen-Begrenzungsblock (215) verbunden ist, eine dritte Klemmnut (216) an dem Boden der Führungsnut (214) vorgesehen ist und eine gerade Anzahl erster Schnallen (217) symmetrisch an dem Klemmteil (213) vorgesehen ist,
**dadurch gekennzeichnet, dass**
die Rückstellsperre umfasst:
eine gerade Anzahl zweiter Schnallen (221), die symmetrisch an einem hinteren Ende der Schiebehülse (22) vorgesehen ist, und eine zweite Klemmnut (222), die an einer Seite des Schiebehülsen-Begrenzungsblocks (215) nahe einer Achse des Innenzylinders (21) vorgesehen ist; wobei
jede zweite Schnalle (221) in der zweiten Klemmnut (222) eingeklemmt ist und eine dritte Schnalle (223) auf einer Seite des Schiebehülsen-Begrenzungsblocks (215) angeordnet ist, welche weit von der Achse des Innenzylinders (21) entfernt ist; wobei
bevor sich die Schiebehülse (22) relativ zu dem Innenzylinder (21) verschiebt, die dritte Schnalle (223) sich in der zweiten Klemmnut (222) befindet, und nachdem die Schiebehülse (22) relativ zu dem Innenzylinder (21) verschoben worden ist, die dritte Schnalle (223) in der dritten Klemmnut (216) eingeklemmt ist, so dass die Schiebehülse (22) nicht in die vorherige Position zurückkehren kann.

2. Die wiederverwendbare anorektale Ligaturvorrichtung nach Anspruch 1, wobei die Druckentlastungskomponente eine Druckentlastungstaste (431), eine Druckentlastungsabdeckung (432) und eine Druckfeder (433) umfasst; und
die Druckentlastungstaste (431) sich aus einem Ende des dritten Anschlusses erstreckt und im dritten Anschluss gleitfähig verbunden ist; und
die Druckentlastungsabdeckung (432) mit dem anderen Ende des dritten Anschlusses verschraubt ist und die Druckfeder (433) zwischen der Druckentlastungstaste (431) und der Druckentlastungsabdeckung (432) angeordnet ist; und
ein Druckentlastungstasten-Dichtungsring (434) zwischen der Druckentlastungstaste (431) und dem dritten Anschluss montiert ist und ein Druckentlastungsabdeckung-Dichtungsring (435) zwischen der Druckentlastungsabdeckung (432) und dem dritten Anschluss montiert ist.

3. Die wiederverwendbare anorektale Ligaturvorrichtung nach Anspruch 1, wobei ein Abluftanschluss der Unterdruckpumpe (42) mit einer Abluftleitung (421) verbunden ist, eine Abluftverbindung (422) an der Abluftleitung (421) montiert ist und ein Rückschlagventil (423) in der Abluftverbindung (422) montiert ist; die Abluftverbindung (422) an dem Boden des Griffs festgeklemmt ist und ein Abluftverbindungsdichtring (424) zwischen der Abluftverbindung (422) und dem Griff montiert ist; und eine Dämpfungshülse (425) eine Außenseite der Unterdruckpumpe (42) bedeckt.

4. Die wiederverwendbare anorektale Ligaturvorrichtung nach Anspruch 1, wobei eine Batterie (51) und die elektrische Steuervorrichtung (5) im Inneren des Griffs montiert sind, ein Batteriefach (16) in dem Griff angeordnet ist und die Batterie (51) lösbar in dem Batteriefach (16) montiert ist; und
die Batterie (51) elektrisch mit der elektrischen Steuervorrichtung (5) und der Unterdruckpumpe (42) jeweils verbunden ist und die elektrische Steuervorrichtung (5) elektrisch mit einem Drucksensor verbunden ist und eine Sonde des Drucksensors in einer Sensorverbindungsleitung (45) angeordnet ist; und
ein Ende der Sensorverbindungsleitung (45) geschlossen ist und das andere Ende der Sensorverbindungsleitung (45) mit einem Vierwegerohr kommuniziert; die Unterdruckpumpe (42) über Drähte mit einer Einschalttaste (52) verbunden ist und die Einschalttaste (52) auf dem Griff montiert ist; die elektrische Steuervorrichtung (5) weiter mit einem Anzeigebildschirm (54) verbunden ist und der Anzeigebildschirm (54) auf dem Griff montiert ist.

5. Die wiederverwendbare anorektale Ligaturvorrichtung nach Anspruch 1, wobei der obere Sitz (33) des Entlüftungsrohrs ein Zylinderverbindungsteil (331) und ein Rohrleitungsverbindungsteil (332) umfasst und das Zylinderverbindungsteil (331) und das Rohrleitungsverbindungsteil (332) durch jeweilige interne Unterdruckkanäle miteinander verbunden sind, das Zylinderverbindungsteil (331) und der Innenzylinder (21) koaxial angeordnet sind, ein hinteres Ende des Innenzylinders (21) in das Zylinderverbindungsteil (331) eingeführt ist und das Rohrleitungsverbindungsteil (332) mit dem Saugmechanismus kommuniziert; und
eine gerade Anzahl von Führungsschienen symmetrisch an zwei Seiten des Zylinderverbindungsteils (331) vorgesehen sind, eine erste Klemmnut (335) an jeder Führungsschiene vorgesehen ist und jede erste Schnalle (217) an dem inneren Zylinderklemmteil (213) in einer entsprechenden ersten Klemmnut (335) festgeklemmt ist.

6. Die wiederverwendbare anorektale Ligaturvorrichtung nach Anspruch 5, wobei an dem Führungsschubblock (32) ein Führungshohlraum vorgesehen ist, der sich nach vorne und hinten erstreckt, und der Führungsschubblock (32) mit dem oberen Sitz (33) des Entlüftungsrohrs durch den Führungshohlraum gleitfähig verbunden ist, eine gerade Anzahl von Schubstangen (322), die mit dem Schiebehülsen-Begrenzungsblock (215) zusammen passen, symmetrisch an einem vorderen Ende des Führungsschubblocks (32) vorgesehen sind; und
am unteren Teil des Führungsschubblocks (32) ein Übertragungsblock vorgesehen ist, der mit dem Auslöseschalter (31) in Kontakt steht, und an jeder Schubstange (322) eine Begrenzungsstufe vorgesehen ist.

7. Die wiederverwendbare anorektale Ligaturvorrichtung nach Anspruch 6, wobei der Griff mit einer linken Entriegelungstaste (13) und einer rechten Entriegelungstaste (14) ausgestattet ist, wobei die linke Entriegelungstaste (13) und die rechte Entriegelungstaste (14) jeweils mit der ersten Schnalle (217) am inneren Zylinderklemmteil (213) zusammen passen und eine L-förmige Haltewand (15) im Griff vorgesehen ist.

8. Die wiederverwendbare anorektale Ligaturvorrichtung nach Anspruch 1, wobei auf dem Griff ein Ladeanschluss und ein Lichtübertragungsanschluss vorgesehen sind, auf dem Ladeanschluss eine staubdichte Schutzabdeckung (17) vorgesehen ist und am Lichtübertragungsanschluss ein Lichtübertragungsteil (18) vorgesehen ist, welches für die Patch-LED geeignet ist, um Licht zu übertragen.

## Revendications

1. Dispositif de ligature ano-rectale réutilisable présentant une forme générale de pistolet, comprenant une poignée et un mécanisme de ligature, dans lequel le mécanisme de ligature est raccordé de manière amovible à la poignée, et un mécanisme de déclenchement pour déclencher le mécanisme de ligature et un mécanisme d'aspiration pour fournir une pression négative pour le mécanisme de ligature sont agencés dans la poignée ; et
le mécanisme de ligature comprend un corps cylindrique interne (21), un manchon poussoir (22) et un composant de serrage de fil (23) pour ligaturer le tissu cible, dans lequel le manchon poussoir (22) est emmanché sur le corps cylindrique interne (21) et est configuré pour être déplacé relativement au corps cylindrique interne (21), et une structure anti-réinitialisation, pour empêcher le manchon poussoir (22) de se réinitialiser après le déplacement, est agencée entre le manchon poussoir (22) et le corps cylindrique interne (21), le au moins un composant de serrage de fil (23) est agencé le long d'une paroi externe du manchon poussoir (22), et le composant de serrage de fil (23) est monté sur le manchon poussoir (22) ; et
le mécanisme de déclenchement comprend un commutateur de déclenchement (31), un bloc-poussoir de guidage (32) et un siège supérieur de tuyau d'aération (33), dans lequel le siège supérieur de tuyau d'aération (33) est raccordé de manière fixe dans la poignée, le bloc-poussoir de guidage (32) est raccordé de manière coulissante au siège supérieur de tuyau d'aération (33), et le commutateur de déclenchement (31) est raccordé en rotation sur la poignée et en raccordement de transmission au bloc poussoir de guidage (32) ; un ressort d'extension (34) est agencé entre le bloc poussoir de guidage (32) et le siège supérieur de tuyau d'aération (33), et le corps cylindrique interne (21) est raccordé de manière amovible au siège supérieur de tuyau d'aération (33) ;
le mécanisme d'aspiration comprend un tuyau à quatre voies (41), une pompe à pression négative (42) et un composant de décompression (43) pour réduire la pression du mécanisme de ligature, dans lequel un premier orifice, un deuxième orifice, un troisième orifice et un quatrième orifice sont prévus dans le tuyau à quatre voies (41) ; et
le premier orifice est mis en communication avec le siège supérieur de tuyau d'aération (33), le deuxième orifice est mis en communication avec un orifice d'aspiration de la pompe à pression négative (42) par l'intermédiaire d'un conduit de raccordement de pompe à pression négative (44), le troisième orifice est monté avec le composant de décompression (43) et le quatrième orifice est raccordé à un conduit de raccordement de capteur (45) ; et
un dispositif de commande électrique (5) pour commander la pompe à pression négative (42) est en outre agencé dans la poignée,
dans lequel une extrémité du corps cylindrique interne (21) est une buse d'aspiration (211), et l'autre extrémité du corps cylindrique interne (21) est une douille (212) ; et
une position de la buse d'aspiration (211) est définie comme étant avant et une position de la douille (212) est définie comme étant arrière ; et
la douille (212) est raccordée de manière fixe à un côté arrière de la buse d'aspiration (211), une partie de serrage (213) est agencée entre la buse d'aspiration (211) et la douille (212), la partie de serrage (213) est proximité d'un côté la douille (212), un nombre pair de rainures de guidage (214), qui vont d'avant en arrière, est prévu symétriquement sur la partie de serrage (213) ; et
chaque rainure de guidage (214) est raccordée de manière coulissante à un bloc limite de manchon poussoir (215), une troisième rainure de serrage (216) est prévue au fond de la rainure de guidage (214), et un nombre pair de premières boucles (217) est prévu symétriquement sur la partie de serrage (213),
**caractérisé en ce que**
la structure anti-réinitialisation comprend :
un nombre pair de deuxièmes boucles (221) étant prévu symétriquement sur une extrémité arrière du manchon poussoir (22), et une deuxième rainure de serrage (222) prévue au niveau d'un côté du bloc limite de manchon poussoir (215) à proximité d'un axe du corps cylindrique interne (21) ; dans lequel
chaque deuxième boucle (221) est serrée dans la deuxième rainure de serrage (222), et une troisième boucle (223) est agencée sur un côté du bloc limite de manchon poussoir (215), le côté étant éloigné de l'axe du corps cylindrique interne (21) ; dans lequel
avant que le manchon poussoir (22) ne se déplace relativement au corps cylindrique interne (21), la troisième boucle (223) est située dans la deuxième rainure de serrage (222), et après que le manchon poussoir (22) a été déplacé relativement au corps cylindrique interne (21), la troisième boucle (223) est serrée dans la troisième rainure de serrage (216) de telle sorte que le manchon poussoir (22) ne puisse pas retourner à la position précédente.

2. Dispositif de ligature ano-rectale réutilisable selon la revendication 1, dans lequel le composant de décompression comprend une touche de décompression (431), un couvercle de décompression (432) et un ressort de compression (433) ; et
la touche de décompression (431) s'étend hors d'une extrémité du troisième orifice et est raccordée de manière coulissante dans le troisième orifice ; et
le couvercle de décompression (432) est raccordé de manière filetée à l'autre extrémité du troisième orifice, et le ressort de compression (433) est agencé entre la touche de décompression (431) et le couvercle de décompression (432) ; et
une bague d'étanchéité de touche de décompression (434) est montée entre la touche de décompression (431) et le troisième orifice, et une bague d'étanchéité de couvercle de décompression (435) est montée entre le couvercle de décompression (432) et le troisième orifice.

3. Dispositif de ligature ano-rectale réutilisable selon la revendication 1, dans lequel un orifice d'échappement de la pompe à pression négative (42) est raccordé à un conduit d'échappement (421), un raccord d'échappement (422) est monté sur le conduit d'échappement (421), et une soupape de non-retour (423) est montée dans le raccord d'échappement (422) ; le raccord d'échappement (422) est serré au bas de la poignée, et une bague d'étanchéité de raccord d'échappement (424) est montée entre le raccord d'échappement (422) et la poignée ; et un manchon d'amortissement (425) couvre un extérieur de la pompe à pression négative (42).

4. Dispositif de ligature ano-rectale réutilisable selon la revendication 1, dans lequel une batterie (51) et le dispositif de commande électrique (5) sont montés à l'intérieur de la poignée, un compartiment de batterie (16) est agencé dans la poignée, et la batterie (51) est montée de manière amovible dans le compartiment de batterie (16) ; et
la batterie (51) est raccordée électriquement au dispositif de commande électrique (5) et à la pompe à pression négative (42), respectivement, et le dispositif de commande électrique (5) est raccordé électriquement à un capteur de pression, et une sonde du capteur de pression est agencée dans un conduit de raccordement de capteur (45) ; et
une extrémité du conduit de raccordement de capteur (45) est fermée, et l'autre extrémité du conduit de raccordement de capteur (45) est en communication avec un tuyau à quatre voies ; la pompe à pression négative (42) est raccordée à une touche d'allumage (52) par l'intermédiaire de fils, et la touche d'allumage (52) est montée sur la poignée ; le dispositif de commande électrique (5) est en outre raccordé à un écran d'affichage (54), et l'écran d'affichage (54) est monté sur la poignée.

5. Dispositif de ligature ano-rectale réutilisable selon la revendication 1, dans lequel le siège supérieur de tuyau d'aération (33) comprend une partie de raccordement de corps cylindrique (331) et une partie de raccordement de conduite (332), et la partie de raccordement de corps cylindrique (331) et la partie de raccordement de conduite (332) sont mises en communication mutuelle par des canaux de pression négative intérieurs respectifs, la partie de raccordement de corps cylindrique (331) et le corps cylindrique interne (21) sont agencés coaxialement, une extrémité arrière du corps cylindrique interne (21) est insérée dans la partie de raccordement de corps cylindrique (331), et la partie de raccordement de conduite (332) est mise en communication avec le mécanisme d'aspiration ; et
un nombre pair de rails de guidage sont agencés symétriquement au niveau de deux côtés de la partie de raccordement de corps cylindrique (331), une première rainure de serrage (335) est prévue sur chaque rail de guidage, et chaque première boucle (217) sur la partie de serrage du corps cylindrique interne (213) est serrée dans une première rainure de serrage (335) correspondante.

6. Dispositif de ligature ano-rectale réutilisable selon la revendication 5, dans lequel une cavité de guidage, pénétrant en va-et-vient, est prévue au niveau du bloc poussoir de guidage (32), et le bloc poussoir de guidage (32) est raccordé de manière coulissante au siège supérieur du tuyau d'aération (33) par l'intermédiaire de la cavité de guidage, un nombre pair de tiges de poussée (322), correspondant au bloc limite de manchon poussoir (215), sont agencées symétriquement au niveau d'une extrémité avant du bloc poussoir de guidage (32) ; et
un bloc de transmission, en contact avec le commutateur de déclenchement (31), est agencé au niveau de la partie inférieure du bloc poussoir de guidage (32), et un épaulement limite est prévu sur chaque tige de poussée (322).

7. Dispositif de ligature ano-rectale réutilisable selon la revendication 6, dans lequel la poignée est équipée d'une touche de libération gauche (13) et d'une touche de libération droite (14), la touche de libération gauche (13) et la touche de libération droite (14) sont respectivement assorties à la première boucle (217) sur la partie de serrage du corps cylindrique interne (213), et une paroi de retenue en forme de L (15) est prévue dans la poignée.

8. Dispositif de ligature ano-rectale réutilisable selon la revendication 1, dans lequel un port de charge et un port de transmission de lumière sont prévus sur la poignée, un couvercle de protection anti-poussière (17) est prévu sur le port de charge, et une pièce de transmission de lumière (18) qui convient au patch LED pour transmettre la lumière est prévue sur le port de transmission de lumière.
